# EUROPEAN PATENT APPLICATION

(11) **EP 2 042 083 A2**
(43) Date of publication of application: **01.04.2009**
(21) Application number: 08009012.9
(22) Date of filing: 15.05.2008
(51) Int. Cl.: A61B 5/00, A61B 5/0205, A61B 5/0408

(54) **Necklace type of detector for electrocardiographic and temperature information**

(30) Priority: 15.05.2007 TW 96117164
(71) Applicant: National Yang-Ming University, Beitou District Taipei City 112 (TW)
(72) Inventor: Kuo, Terry B.J., Ji-an Township Hualien County (TW); Yang, Ching-Hsiu, Ji-an Township Hualien County (TW)
(74) Representative: TER MEER - STEINMEISTER & PARTNER GbR

(57) **Abstract**

The present invention provides an non-adhering electrocardiographic and temperature signal detector comprising: (a) an electrocardiographic detecting working electrode, an electrocardiographic reference electrode and a temperature sensor connected together by a conduction chain; (b) a signal processing unit for processing those signals obtained from said electrode or sensor, (c) a wireless transceiver for sending and receiving processed signal of electrocardiographic to the receiver at a far end unit or receiving data from the far end unit; and (d) a power supply supplying power to the operation of the detector. The detector improves the uncomfortable of conventional adhering detector and increase the willing of a use due to the aesthetic outlook of the detector and convenient to detect. Accordingly, the electrocardiographic and body temperature gas an opportunity to apply home care device.

## Description

### FIELD OF THE INVENTION

Through the integration of technologies of micro physiological signal wireless transmission detector, synchronized transmission and receiving technologies, synchronized storing and recording technologies, physiological signal detection and analytical technologies and sleeping analytical algorithm, a physiological signal recording system having no lead wire, easy to operate, monitor and detection at any time and accurate analysis can be achieved. The system can be used on the evaluation of sleeping quality for normal person and patients having diseases and applicable for diagnosis of long term patients having sleep obstacle. The system can be used on efficiency valuation of long term sleeping pills and other pharmaceuticals with respect to sleeping and side effect on self-neurological function, and health care with respect to sleep and the alteration of self neurological function, and other alternative of various health product, on the valuation on old age body, and sleeping problem of newborn babies.

### DESCRIPTION OF RELATED ART

Generally, research in medical science is classified into two major areas, i.e., structural and functional. With respect to functional, various methods have been exploited to determine the function of each organ and tissues.

Recently, great progress is found in the growth of functional medical study. There are lots of techniques established for physiological signal detection. But in order to get more accurate information, an invasive method for physiological signal collection is employed. For example, heart catch (also called cardiac catheterization), a method shown blood vessels of the heart and the inside of the heart as it pumps, needs to put a catheter into artery then guided into the heart. It increases the accuracy for diagnosis and also increases the risk in the course of a surgery and the patient should endure the pain.

Comparatively, non-invasive method is much safer as compared to an invasive method. This is due to the indirect detection method which cannot obtain accurate signal for medical use.

Fortunately, in view of the development of computer science, powerful software and hardware are used to improve signal processing ability of the non-invasive detector. Heart rate variability (HRV) analysis (Anonymous 1996, Circulation 93: 1043-1065*.*) represents a good example for non-invasive diagnosis, which detects the skin surface voltage variability and processes those signals to estimate of the function of the sympathetic and parasympathetic autonomic nervous systems in patients. This technique has been successfully applied on analysis of cardiovascular fluctuations during pentobarbital anesthesia (Yang et al. 1996, American Journal of Physiology 270:H575-H582*.),* brain death determining (Kuo et al. 1997, American Journal of Physiology 273: H1291-H1298*.),* prediction of patient outcome in an intensive care unit (Yien et al. 1997, Critical Care Medicine 25: 258-266*.*), and observation the effect of aging on gender differences in neural control of heart rate (Kuo et al. 1999, American Journal of Physiology 277: H2233-H2239*.),* but consider to the convenience of those non-invasive technique and the feeling of patients, there are room for improvement.

### Critical of Non-Invasive Diagnosis Technology Development

The non-invasive diagnosis technique mainly consists two parts, the first one is a detector and the other one is a signal processing unit. The detector is the most important part in this field for the reason that, without a suitable detector, no accurate signal could be obtained and the further, signal procession is useless, or if the detector is difficult to apply on a patient, and the patient feels uncomfortable to use the invasive one. Thus it is important to develop a non-invasive diagnosis technique for which a detector is accurate, comfortable to be applied on a patient and is user friendly.

The Importance of Establishing A Wireless Physiological Signal Detection System
(1) Presently, the long-term physiological signal detection system is based on the wire-transmission technique. When a patient needs to use this system, plurality electrodes are employed on his skin, and the signals collected by those electrodes will be transmitted through connected wires to a signal processing unit to be further processed such as analog to digital conversion and signal amplification. Generally, a period of time is used in collect those required signals, and the electrodes and connected wires would cause uncomfortable and hindrance to the patient. Besides, the unfriendly interface of this system needs a professional technician to operate the machine. Thus, this diagnosis system is generally not easy to apply daily.
(2) There are numerous of systems where measuring is not easy. Very expertise professionals or technicians are needed, and the professionals and technicians require long hour training and only the trained professional or technicians could implement physiological phenomena detection. Thus these systems are not easy to be operated.
(3) Recently, some companies have developed new wireless systems to transmit detected signals. Those systems generally comprise three parts, one the first part, detecting electrodes, the second part, a signal processor, and the third part, a far-end signal analysis/storage machine. The electrodes detect physiological signals, and deliver the signals through wires to the signal processor to process to analog-to-digital conversion and amplification, and then transmit those signals to the far-end signal analysis/storage machine. This system has been successfully applied on clinical uses and gives a hint that wireless technique can be applied on this area. However, this system still can not integrate the electrode and signal processor together, only when those two parts are built separately and wires are used to connect them, thus this limitation increases some side-effects accompanied by the wires used, such as noise and too heavy to carry.

### Troubleshooting to be solved

The great progress of diagnosis technology allows automatic nervous activity to be quantified through the processing of the electrocardiography (ECG) signals. In order to obtain more specific physiological signals most of the electrodes used were disposable patch-form which adheres onto a patient's skin, but these sticky electrodes somehow cause skin allergy, and the non-reusable electrodes increase the burden to the user's. Thus, the user is reluctant to use them regularly or for long term.

If a non-invasive detector become a daily-use necklace on wearing a subject and such detector is designed to express an article with beautiful outlook together with a user can use the detector under quiet condition or can fix the detector for a duration, such detector can enhance the practicability of non-invasive technology and can be worn a long time to achieve long term care.

### SUMMARY OF THE INVENTION

The present invention provides an non-adhering electrocardiographic and temperature signal detector comprising: (a) an electrocardiographic detecting working electrode, an electrocardiographic reference electrode and a temperature sensor connected together by a conduction chain; (b) a signal processing unit for processing those signals obtained from said electrode or sensor, (c) a wireless transceiver for sending and receiving processed signal of electrocardiographic to the receiver at a far end unit or receiving data from the far end unit; and (d) a power supply supplying power to the operation of the detector.

### BRIEF DESCRIPTION OF THE DRAWINGS

Figure 1 shows an embodiment of necklace type of electrocardiographic and temperature signal detector of the present invention, wherein the electrocardiographic reference electrode is an independent conductive chain.

Figure 2 shows an embodiment of necklace type of electrocardiographic and temperature signal detector of the present invention, wherein the electrocardiographic reference electrode and the conductive chain are formed as one unit.

Figure 3 is a schematic view showing the detector being wore by user.

Figure 4 is a schematic view showing the circuit design of the detector of the present invention.

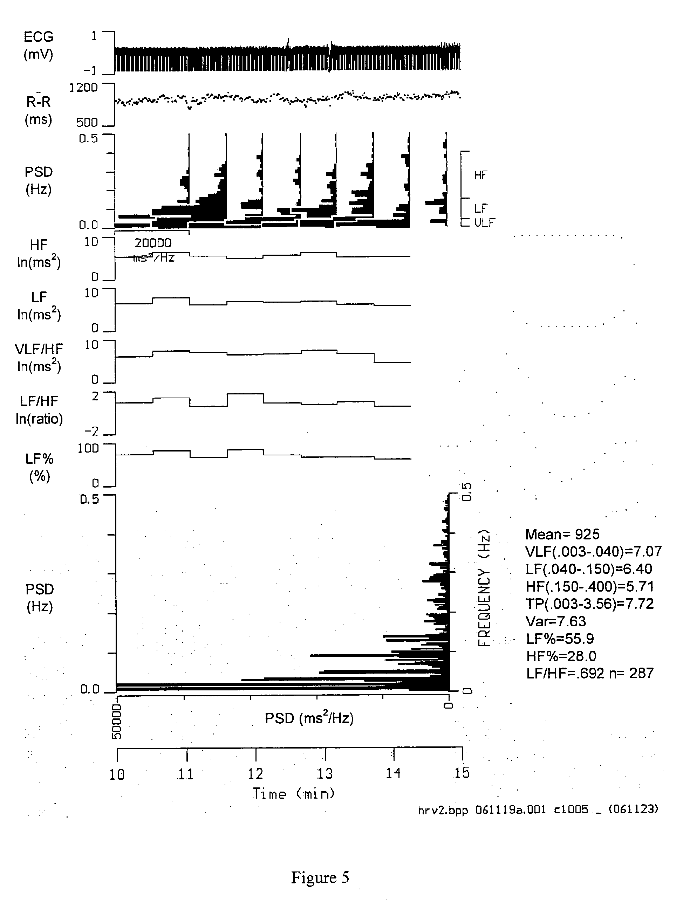

Figure 5 illustrates heart beat changes information by spectrum analysis of electrocardiographic signal detected by the detector of the present invention.

### DETAIL DESCRIPTION OF THE INVENTION

The present invention relates to an necklace type of non-adhering electrocardiographic and temperature signal detector comprising: (a) an electrocardiographic detecting working electrode, an electrocardiographic reference electrode and a temperature sensor connected together by a conduction chain; (b) a signal processing unit for processing those signals obtained from said electrode or sensor, (c) a wireless transceiver for sending and receiving processed signal of electrocardiographic to the receiver at a far end unit or receiving data from the far end unit; and (d) a power supply supplying power to the operation of the detector.

The electrocardiographic electrode and the electrocardiographic reference electrode are constructed into a two-electrode system. The electrocardiographic electrode detects surface voltage of a user's skin and electrocardiographic signal is compared with the surface voltage of the reference electrode at fixed potential. The detection can be obtained by direct contact with the skin or indirect sensing of the skin. In this invention, the function of the reference electrode is used as a reference for amplified electrocardiographic signals. The chain used in this invention contains a conductive wire and is connected with a pendant, and the conductive chain and the pendant can be of various shapes.

The electrocardiographic working electrode and the electrocardiographic reference electrode of this invention are placed separately on the conductive chain and on a pendant connected to the chain, wherein the electrode placed on the chain part is the chain per se or is a conductive plate on the chain. The electrode placed on the pendant is a conductive outer shell of said pendant or is placed at the interior of the conductive or non-conductive pendant. The conductive outer shell is made by metal or by plastic having processed on the surface thereof. For instance by electroplating or coating with conductive paint, graphite or carbon fiber or other conductor.

In a preferred embodiment, the method of detecting by the detector is thermal resistance effect, thermistor, thermocouple, or any non-invasive electric-thermometer.

The present signal processing unit of this invention comprises the detector wherein the signal processing unit comprising: (a) a signal amplifier; for amplify the electronic signal of the obtained electrocardiographic or temperature, (b) an electronic filter for screening the distorted signal detected; and (c) an analog-to-digital converter (ADC) to convert the analog signal to digital signal.

The wireless transceiver of this invention transmits signals through radio wave interface or infrared interface, wherein the radio wave interface is wireless, WLAN, INTEREST RATE, Bluetooth, RFID, GSM, PHS, CDMA or any other radio wave interface that can be used for electronic signal transmission.

The power supply is a storage battery. Further the power supply is batteries. The signal processing unit of the present invention, wireless transceiver and the power supply are altogether or separately provided in one or more than one pendant.

The detector further comprises a data storage unit for storing electronic physiological signal after being processed by the signal processing unit. In further, the data storage unit is Random Access Memory (RAM) or any device for storing electrical signal.

The size of the detector of the present invention likes a common necklace. The detector adopts non-adhering design to facilitate long-term wearing and to mitigate uncomfortable feeling when eletrocardiographic signal. Meanwhile, the detector could be designed as an article with beautiful and elegant outlook to increase the acceptability of a customer.

Because the electrocardiographic signal on body surface is very weak and signal strength becomes gradually weak from heart to limb ends, the detector of the present invention is always worn on neck. The use of the pendant contacts with chest to detect stronger electrocardiographic signal on chest. However, the detector could be applied to other position such as limbs.

### EXAMPLE

The examples are only illustrative of the present invention and explain the various advantages of different embodiments. The examples should not be limited to such embodiments.

### 1. Detector

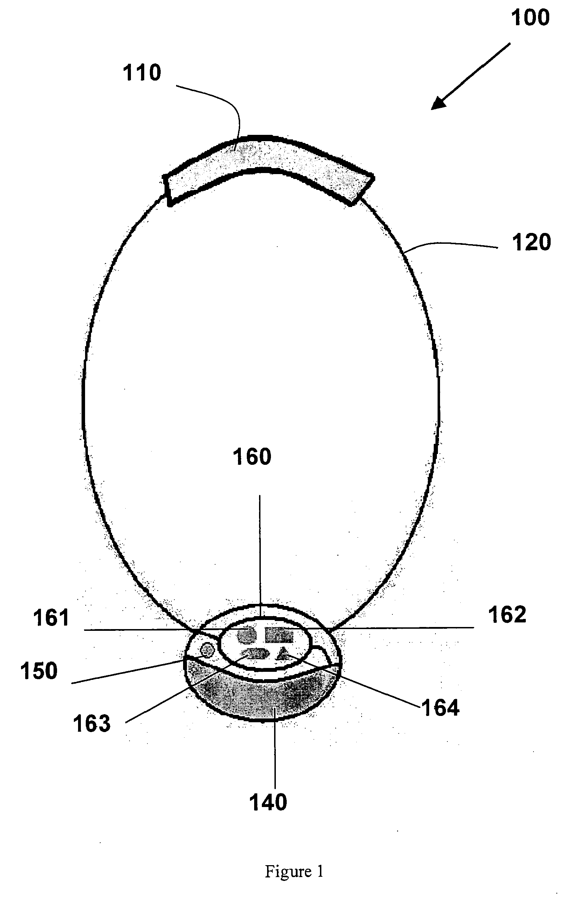

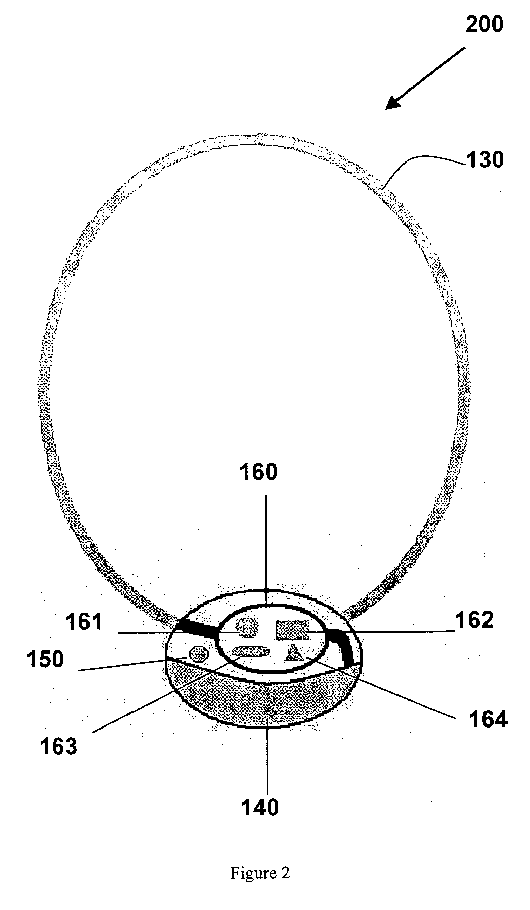

Referring to FIG. 1, there is shown one embodiment of present invention, which is a non-adhering stuck necklace-type detector 100 having an electrocardiographic working electrode 140, a temperature sensor 150, a computing center 160 (contains signal processing unit 161, data storage unit 163, and wireless transceiver 164), a battery 162, and a reference electrode 110, wherein the former four components are placed within a pendant and the last component is a metal plate placed on a chain connecting the sensor 150. Figure 2 is another preferred embodiment of this invention which is slightly modification as compared to Figure 1 with respect to the reference electrode 130. The reference electrode 130 in Figure 2 is integrated into the chain. In this example, the working electrode 140 is an induction coil placed within the pedant.

### 2. Usage

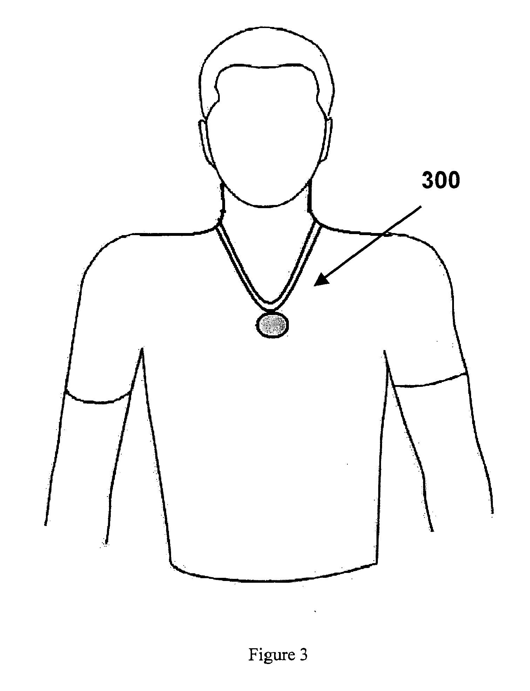

As shown in Figure 3, this detector 300 is worn on user's neck and the pendant hangs in front of chest. To perform a measurement, the user is asked to keep at a steady posture and the detector is in contact with the skin of the user for a few seconds. Then the electrocardiographic signal is obtained from detecting the chest surface the voltage of the surface of the chest by the working electrode 140, and the temperature information will be collected by the temperature sensor 150.

### 3. Collection of electrocardiographic signal

To improve the convenience and reliability of electrocardiography measurement, a two-electrode system is adopted, but this system has more noise problems than a three-electrode system. Thus, we need some appropriate filter circuit and opto-coupler to overcome this defect. For example, in this invention an amplification device (a R.O.C. patent of the same inventor, patent No. 149299, application date 1998/01/15) is used to amplify electrocardiographic signal detected by the working electrode and to obtain a useful wave pattern of the signal-to-noise ratio.

### 4. Body temperature measurement

The present invention has another function on body temperature measurement. The detection principle is the same as any commercial electric thermometer that detects a voltage change, a resistance change or a current change on the inner circuit of the sensor caused by heat transmission, and converts this information into electric signals

### 5. Proceeding, storage and transmitting of signal ( micro-compute, filter and amplify)

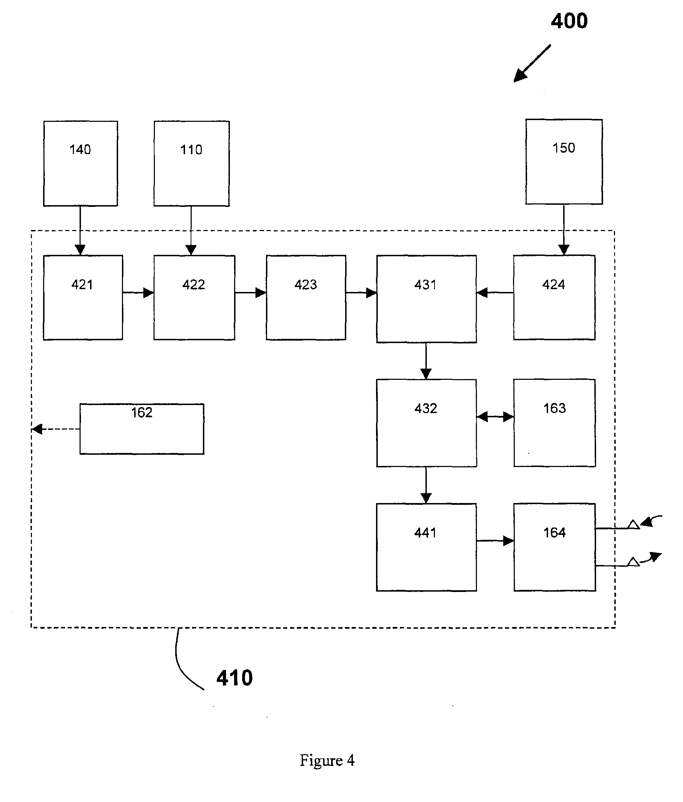

Figure 4 shows a diagram of the circuit design 400 of the present invention. The electrocardiographic signals obtained from the working electrode 140 first passes through an input filter 421 to enhance signal-to-noise ratio. Then, the signals are amplified by an amplifier 422 which uses the reference electrode 110 as a standard, and then the signals pass through an outpour filter 423 in order to facilitate the sampling. The body temperature signals also pass through a filter/amplify unit 424 to be filtered and amplified. These two signals are then converted into digital signals after the signals pass through an analog-to-digital converter (ADC) 431 and are processed in a micro arithmetic logic unit 432, and then either stored in a memory unit 163 or pass through a modulator/demodulator 441 to further transmit the signals to a far-end computer through the wireless transceiver 164. The wireless transceiver 164 also can receive the feedback signal sent from the far-end receiver. A power supply 162 is used to supply power to this signal processing unit 410.

### Analysis of signal

### 1. Signal receipt

There are many useful wireless interfaces for signal transmission at such as radio wave and infrared rays. In this example Infrared Data Association (IrDA) interface was adopted for signals transmission and the far-end computer had an infrared port (IR port) to receive the signals sent by this detector and had the ability to process those analysis listed below:

### 2. Identification of heart beat

The computer program for HRV analysis was described in our previous publication *(*Kuo et al. 1999, American Journal of Physiology 277: H2233-H2239*.*). In the QRS identification procedure, the computer first detected all peaks of the digitized ECG signals using a spike detection algorithm similar to general QRS detection algorithms. Parameters such as amplitude and duration of all spikes were measured so that their means and standard deviations (SD) could be calculated as standard QRS templates. Each QRS complex was then identified, and each ventricular premature complex or noise was rejected according to its likelihood in standard QRS templates. The R point of each valid QRS complex was defined as the time point of each heart beat, and the interval between two R points (R-R interval) was estimated as the interval between current and latter R points. In the R-R interval rejection procedure, a temporary mean and SD of all R-R intervals were first calculated for standard reference. Each R-R interval was then validated: if the standard score of an R-R value exceeded 3, it was considered erroneous or nonstationary and was rejected. The average percentile of R-R rejection according to this procedure was 1.2%. The validated R-R values were subsequently resampled and interpolated at the rate of 7.11 Hz to accomplish the continuity in time domain.

### 3. Frequency-domain analysis

Frequency-domain analysis was performed using the nonparametric method of fast Fourier transform (FFT). The direct current component was deleted, and a Hamming window was used to attenuate the leakage effect. For each time segment (288 s, 2,048 data points) the algorithm estimated the power spectral density on the basis of FFT. The resulting power spectrum was corrected for attenuation resulting from the sampling and the Hamming window. The power (TP) spectrum was subsequently quantified into various frequency-domain measurements as defined in Table 1. In particular, LF was normalized by the percentage of total power except for VLF (total power VLF) to detect sympathetic influence on HRV (LF%). A similar procedure was also applied to HF (HF%). All HRV parameters were expressed in original, square root, and natural logarithmic form to demonstrate and correct possible skewness.

### 4. DATA decipherment

The data were deciphered according to the report published by American Heart Association Inc. and European Society of Cardiology (Anonymous 1996, Circulation 93: 1043-1065*,* and simultaneously published in European Heart Journal (1996) 17, 354-381*.*), and also the inventors' previously publications (Kuo et al. 1999, American Journal of Physiology 277: H2233-H2239*;* Kuo et al. 1997, American Journal of Physiology 273: H1291-H1298*;* Yang et al. 2000, American Journal of Physiology-Heart and Circulatory Physiology 278:H1269-1273*;* Yien et al. 1997, Critical Care Medicine 25: 258-266). The HF and TP value were used for measuring the activity of cardiac sympathetic nerve, LF/HF ratio was used for measuring the activity of cardiac parasympathetic nerve, and LF value was a target for estimating the function of sympathetic and parasympathetic nerves.

One skilled in the art readily appreciates that the present invention is well adapted to carry out the objectives of the present invention and to obtain the advantages mentioned, as well as those inherent therein. The necklace-type non-adhering detector for electrocardiographic and temperature information measurement being representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations, which are not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. An non-adhering electrocardiographic and temperature signal detector comprising: (a) an electrocardiographic detecting working electrode, an electrocardiographic reference electrode and a temperature sensor connected together by a conduction chain; (b) a signal processing unit for processing those signals obtained from said electrode or sensor, (c) a wireless transceiver for sending and receiving processed signal of electrocardiographic to the receiver at a far end unit or receiving data from the far end unit; and (d) a power supply supplying power to the operation of the detector.

2. The detector according to claim 1, wherein the electrocardiographic detecting electrode and the reference electrode are constructed into a two-electrode system, the former detects the surface voltage of user's skin and the latter provides a reference voltage, an electrocardiographic signal obtained by comparing the surface voltage with that of the reference voltage.

3. The detector according to claim 2, wherein the electrocardiographic detecting electrode detects the surface voltage of the user's skin directly or indirectly by detecting the potential signal.

4. The detector according to claim 1, wherein the reference electrode is employed as reference with respect to detection and amplification of electrocardiographic signals.

5. The detector according to claim 1, wherein the electrocardiographic detecting electrode and the reference electrode are respectively mounted on a ring conductive chain and a pendant linked to said chain.

6. The detector according to claim 5, wherein the electrode on the conductive chain is the conductive chain per se or a conductive plate on the conductive chain and/or the electrode on the pendant is the conductive outer shell of the pendant or the interior of the conductive or non-conductive outer shell of the pendant.

7. The detector according to claim 6, wherein the conductive outer shell of pendant is metal or processed on the outer surface of plastic.

8. The detector according to claim 7, wherein the plastic outer shell is processed by electroplating of metal or coating with conductive paint, graphite, carbon fiber or other conductor.

9. The detector according to claim 1, wherein the temperature sensor temperature by means of thermal resistance effect, thermistor, thermocouple, or any non-invasive electric-thermometer.

10. The detector according to claim 1, wherein the signal processing unit comprising: (a) a signal amplifier; for amplify the electronic signal of the obtained electrocardiographic or temperature, (b) an electronic filter for screening the distorted signal detected; and (c) an analog-to-digital converter (ADC) to convert the analog signal to digital signal.

11. The detector according to claim 10, wherein the electronic filter is of the electrocardiographic or temperature selected from the group consisting of low-pass filter, high-pass filter, band-pass filter, and band-stop filter.

12. The detector according to claim 1, wherein the radio wave interface is wireless WLAN, infrared, bluetooth, RFID, GSM, PHS or CDMA or other wireless transmission interface.

13. The detector according to claim 1, wherein the signal processing unit, the wireless transceiver and the power supply are contained altogether or separately in one or more pendant.

14. The detector according to claim 1, which further comprising a data storage unit for storing electronic physiological signal after being processed by the signal processing unit.

15. The detector according to claim 1, wherein the power supply is a storage battery.

16. The detector according to claim 5, wherein the conductive chain and the pendant are of various shapes.

17. The detector according to claim 14, wherein the data storage unit is any electrical signals storage device.

18. The detector according to claim 1, which is worn on user's neck, wrists or limbs.
